Europäisches Patentamt

European Patent Office

Office européen des brevets

(19)

(11) Numéro de publication : **0 055 637**
**B1**

(12) **FASCICULE DE BREVET EUROPÉEN**

(51) Int. Cl.⁴ : **G 01 N 25/04**

(45) Date de publication du fascicule du brevet :
20.02.85

(21) Numéro de dépôt : 81401735.6

(22) Date de dépôt : 29.10.81

(54) **Dispositif pour mesurer la déformation d'un matériau sous l'effet de la chaleur et son application à la détermination du pouvoir mouillant des brais.**

(30) Priorité : 30.12.80 FR 8027742

(43) Date de publication de la demande :
07.07.82 Bulletin 82/27

(45) Mention de la délivrance du brevet :
20.02.85 Bulletin 85/08

(84) Etats contractants désignés :
AT BE CH DE FR GB IT LI LU NL SE

(56) Documents cités :
GB-A- 514 484
GB-A- 1 246 303

(73) Titulaire : **HUILES, GOUDRONS ET DERIVES**
**Tour Aurore Place des Reflets**
**F-92080 Paris la Defense Cédex no. 5 (FR)**

(72) Inventeur : **Couderc, Pierre**
**78 rue Emile Zola**
**F-62400 Bethune (FR)**

(74) Mandataire : **Dubost, Thierry**
**SOCIETE CHIMIQUE DES CHARBONNAGES Service**
**Propriété Industrielle B.P. 57**
**F-62670 Mazingarbe (FR)**

Jouve, 18, rue St-Denis, 75001 Paris, France

## Description

La présente invention concerne un dispositif pour mesurer la déformation d'un matériau sous l'effet de la chaleur et son application à la détermination du pouvoir mouillant des brais.

On connaît des méthodes permettant de mesurer la température à laquelle un matériau, soumis à l'action de la chaleur, passe de l'état solide à l'état pâteux puis à l'état liquide. Ainsi on détermine pour les matériaux thermoplastiques le point de ramollissement bille et anneau ou le point de fusion Durran ou le point de ramollissement Kraemer-Sarnow (KS). Mais ces méthodes ne donnent pas de renseignement sur le comportement du matériau pendant la période au cours de laquelle il est chauffé.

Or il peut être intéressant de connaître l'allure de la variation de la forme d'un matériau soumis à l'action de la chaleur, en particulier son évolution par rapport à un support auquel il est associé et qui, lui, n'est pas sensible à l'action de la chaleur. Dans le domaine particulier des brais liants pour électrodes il est intéressant de connaître le pouvoir mouillant dudit brai qui permet d'apprécier sa valeur d'usage pour la fabrication d'électrodes. En particulier il est intéressant de connaître la température à laquelle un brai donné est complètement adsorbé par un coke donné et, complémentairement, de savoir comment s'effectue cette adsorption, en particulier comment le brai en fusion pénètre les pores du coke.

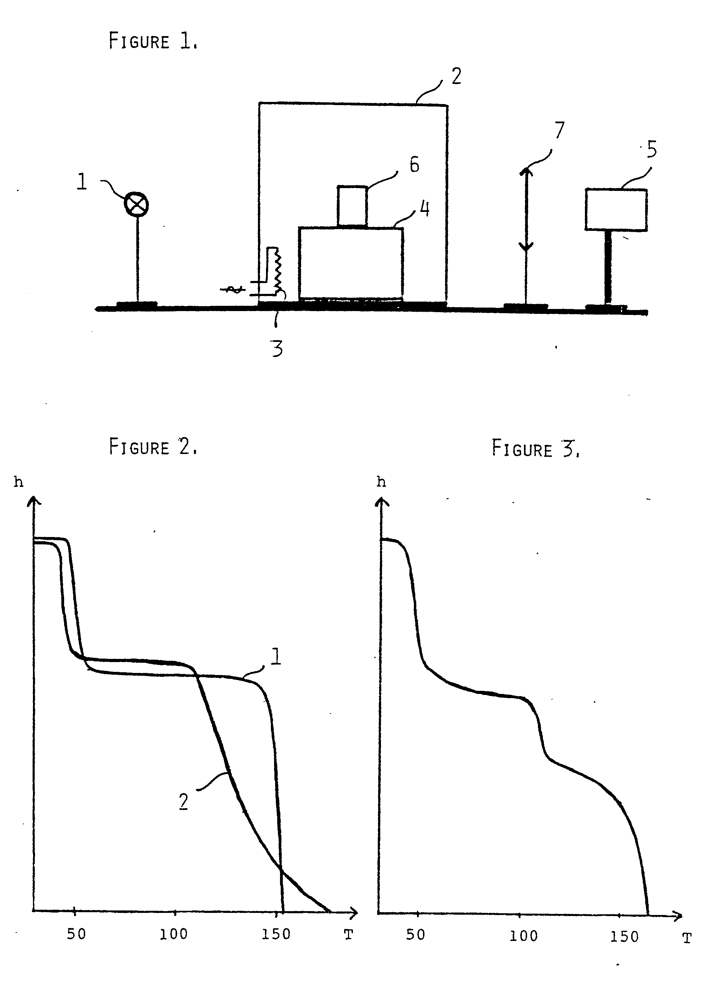

Un premier objet de l'invention, illustré par la figure 1, concerne un dispositif pour mesurer la déformation d'un matériau (6) sous l'effet de la chaleur. Ce dispositif comprend :
— une source lumineuse (1),
— une enceinte (2) au moins partiellement transparente, munie d'un dispositif de chauffage (3) programmable en fonction du temps et d'un support poreux (4) destiné à recevoir le matériau (6),
— un récepteur photoélectrique (5), et
— une première lentille optique (7) permettant de former l'image du matériau (6) sur le récepteur photoélectrique (5),
la source lumineuse (1), le support poreux (4), le récepteur photoélectrique (5) et la première lentille optique (7) étant alignés optiquement.

Accessoirement on pourra intercaler entre la source lumineuse (1) et l'enceinte (2), un diaphragme et/ou une deuxième lentille optique donnant de la source lumineuse (1) un faisceau de lumière parallèle.

Accessoirement le support poreux (4) peut reposer sur un socle qui, soit repose au fond de l'enceinte (2), soit est suspendu sur l'ouverture supérieure de l'enceinte (2).

Le matériau (6) est rigide à température ambiante. Il a avantageusement une forme régulière, de préférence cylindrique ou parallélépipédique.

La nature de la source lumineuse (1) est fonction de la nature du récepteur photoélectrique (5).

Si celui-ci est constitué d'un tube photomultiplicateur, muni d'une fente verticale sur laquelle se formera l'image du matériau (6) donnée par la première lentille (7), il est nécessaire que la source lumineuse soit d'intensité constante et émette une lumière de longueur d'onde compatible avec les caractéristiques du photomultiplicateur. Dans ce cas on peut donc utiliser une source classique (lampe à filament alimenté sous tension stabilisée) ou une source à laser. Si le récepteur photoélectrique est constitué d'une barrette verticale de microphotodiodes, il n'est pas nécessaire que la source lumineuse soit d'intensité constante, ni qu'elle soit monochromatique, les microphotodiodes fonctionnant, ainsi qu'il sera précisé ci-dessous, selon le principe du « tout ou rien ». Mais il est nécessaire, dans ce cas, que l'intensité lumineuse émise par la source soit suffisante pour saturer les microphotodiodes.

L'enceinte (2) doit être au moins partiellement transparente de façon à laisser passer le faisceau lumineux provenant de la source (1). Elle peut être entièrement transparente et être constituée, par exemple, d'un cylindre de verre dont l'axe est perpendiculaire au faisceau lumineux et au plan formé par le support du dispositif. Elle peut également être partiellement transparente et être constituée, par exemple, d'une cuve parallélépipédique dont les deux parois parallèles au faisceau lumineux sont opaques et dont les deux parois perpendiculaires au faisceau lumineux sont transparentes. Les parois transparentes de l'enceinte se faisant ainsi vis-à-vis sont avantageusement en matériau indéformable à la chaleur. Elles peuvent être en verre, par exemple du type Pyrex.

La source lumineuse (1) peut être située à l'intérieur ou à l'extérieur de l'enceinte (2). Si elle est située à l'intérieur de l'enceinte, cette dernière pourra ne comprendre qu'une seule fenêtre pour le passage du faisceau lumineux.

Selon le mode de réalisation de l'invention représenté par la figure 1, la source (1), le support poreux (4), le récepteur photoélectrique (5) et la première lentille optique (7) sont alignés géométriquement. Selon un autre mode de réalisation ces éléments ne sont pas alignés géométriquement ; mais alors le dispositif comprend un organe de réflexion situé sur le parcours du faisceau lumineux provenant de la source (1) avant ou après son occultation par le matériau (6). L'organe de réflexion peut être un miroir ou un prisme à réflexion totale, situé par exemple à l'intérieur de l'enceinte.

Le support poreux (4), de porosité comprise entre 0,1 et 0,9 (rapport du volume des pores au volume total), peut être constitué, par exemple, par un lit de poudre contenu dans une nacelle ou par une pastille en matériau fritté. La poudre contenue dans une nacelle a, par exemple, une granulométrie comprise entre 10 et 1 000 μm. La pastille en matériau fritté a, par exemple, un diamètre de pores compris entre 10 et 200 μm.

Le dispositif selon l'invention fonctionne de la manière suivante. Le matériau (6) est mis en place sur le support poreux (4), l'enceinte (2) étant à une température inférieure à la température à laquelle le matériau (6) commence à se déformer. La source lumineuse (1) est mise sous tension, la première lentille optique (7) est réglée de façon à former l'image du matériau (6) sur le récepteur photoélectrique (5). On vérifie qu'au moins une partie du support poreux (4) intercepte partiellement le faisceau lumineux. Lorsque le récepteur photoélectrique (5) est constitué d'une barrette de microphotodiodes on règle finement le dispositif en s'aidant d'un compteur indiquant le nombre de diodes non illuminées. On chauffe l'enceinte (2) selon une loi T = f (t) connue (T représente la température et t le temps). A partir d'une température donnée, le matériau (6) commence à se déformer et la quantité de lumière reçue par le récepteur photoélectrique (5) augmente proportionnellement à la diminution de la hauteur (h) du matériau (6) posé sur le support poreux (4). Le récepteur photoélectrique (5) émet alors une tension proportionnelle à cette diminution de hauteur. Cette tension permet, par l'intermédiaire d'un enregistreur, de tracer la courbe h = f (t), pour une loi de chauffe connue ou, si l'enregistreur est couplé au programmateur de température de l'enceinte, la courbe h = f (T).

Un perfectionnement au dispositif permet de tracer à tout moment le contour du matériau (6) en cours de déformation. Pour cela le récepteur photoélectrique (5) est monté mobile transversalement par rapport au faisceau lumineux et peut ainsi à tout instant balayer la zone où se forme l'image du matériau (6). Le déplacement doit être suffisamment rapide pour permettre de prendre un cliché quasiment instantané des contours du matériau (6) en cours de déformation, à une température donnée. Cette possibilité donne des renseignements intéressants quant à l'angle formé entre la base du matériau (6) et le support poreux (4), indice du mouillage ou du non-mouillage de l'un par l'autre. On peut utiliser simultanément un enregistreur XY pour dessiner le contour de l'échantillon et un enregistreur h = f (t) ou h = f (T) pour mesurer la plus grande hauteur du matériau (6).

Le dispositif selon l'invention permet d'effectuer des mesures fiables et reproductibles, ne nécessitant pas de contrôle permanent de la part d'un opérateur.

L'invention est applicable à tout matériau se déformant sous l'effet de la chaleur tel que les résines novolaques, les brais et les matériaux thermoplastiques suffisamment opaques.

Un second objet de l'invention concerne une application du dispositif décrit ci-dessus à la détermination du pouvoir mouillant des brais.

Dans cette application, le support poreux (4) est par exemple un lit de poudre de coke contenu dans une nacelle, ou une pastille de verre fritté. Selon le mode opératoire décrit ci-dessus, la mesure est interrompue quand l'échantillon de brai a entièrement été adsorbé par le support poreux (4).

Les exemples suivants sont donnés à titre d'illustration de l'invention :

Exemples 1 et 2

On a utilisé comme support poreux (4) un lit de poudre de coke de granulométrie 40-400 microns dont 88% entre 80 et 125 microns, et comme matériaux (6) deux pastilles de brai ayant sensiblement les mêmes caractéristiques physico-chimiques mais ayant un comportement différent lors de la fabrication d'électrodes : l'échantillon n° 2 a un mauvais comportement contrairement à l'échantillon n° 1. A l'aide du dispositif selon l'invention on a obtenu les courbes de la figure 2 représentant la hauteur des pastilles de brai en fonction de la température. On s'aperçoit que la courbe représentative de l'échantillon de brai n° 2 montre une traînée indiquant que certains de ses constituants sont plus difficilement adsorbés par le lit de poudre de coke.

Exemple 3

On a utilisé comme support poreux (4) une pastille de verre fritté dont le diamètre des pores est compris entre 90 et 150 μm, et comme matériau (6) une pastille de résine formo-phénolique du type novolaque obtenue par condensation de phénol et de formol en milieu acide. Cette résine a un point de ramollissement Durran de 85 °C. La figure 3 montre l'allure de la courbe h = f (T) obtenue à l'aide du dispositif selon l'invention. Un premier palier correspond à la transformation en goutte de la pastille de résine. La température au centre de ce palier est la température de ramollissement. La partie ultérieure de la courbe montre comment l'échantillon est adsorbé par le support poreux (4).

**Revendications**

1. Dispositif pour mesurer la déformation d'un matériau (6) sous l'effet de la chaleur caractérisé en ce qu'il comprend :
— une source lumineuse (1),
— une enceinte (2) au moins partiellement transparente, munie d'un dispositif de chauffage (3) programmable en fonction du temps et d'un support poreux (4) destiné à recevoir le matériau (6),
— un récepteur photoélectrique (5) et
— une première lentille optique (7) permettant de former l'image du matériau (6) sur le récepteur photoélectrique (5),
la source lumineuse (1), le support poreux (4), le récepteur photoélectrique (5) et la première lentille optique (7) étant alignés optiquement.

2. Dispositif selon la revendication 1, caractérisé en ce qu'il comprend en outre entre la source lumineuse (1) et l'enceinte (2), un diaphragme et/ou une deuxième lentille optique.

3. Dispositif selon l'une des revendications 1 et 2, caractérisé en ce que la source lumineuse (1) est située à l'intérieur de l'enceinte (2).

4. Dispositif selon l'une des revendications 1 à 3, caractérisé en ce que la source lumineuse (1), le support poreux (4), le récepteur photoélectrique (5) et la première lentille optique (7) ne sont pas alignés géométriquement et en ce que le dispositif comprend un organe de réflexion sur le parcours du faisceau lumineux provenant de la source (1) avant ou après son occultation par le matériau (6).

5. Dispositif selon l'une des revendications 1 à 4, caractérisé en ce que le récepteur photoélectrique (5) est un tube photomultiplicateur.

6. Dispositif selon l'une des revendications 1 à 4, caractérisé en ce que le récepteur photoélectrique (5) est une barrette de microphotodiodes.

7. Dispositif selon l'une des revendications 1 à 6, caractérisé en ce que le récepteur photoélectrique (5) est monté mobile transversalement par rapport au faisceau lumineux provenant de la source lumineuse (1).

8. Dispositif selon l'une des revendications 1 à 7, caractérisé en ce que le support poreux (4) est un lit de poudre de coke, contenu dans une nacelle.

9. Dispositif selon l'une des revendications 1 à 7, caractérisé en ce que le support poreux (4) est une pastille de verre fritté.

10. Application du dispositif selon l'une des revendications 1 à 9 à la détermination du pouvoir mouillant des brais, sur la base du traçage du contour de ces brais en cours de déformation.

## Claims

1. Device for measuring the deformation of a material (6) under the influence of heat, characterised in that it comprises :
— a source of light (1) ;
— an at least partly transparent chamber (2), equipped with a heating device (3), which can be programmed as a function of time, and with a porous support (4), designed to receive the material (6) ;
— a photoelectric receiver (5) ;
— and a first optical lens (7), enabling the image of the material (6) to be formed on the photo-electric receiver (5), the source of light (1), the porous support (4), the photo-electric receiver (5) and the first optical lens (7) being optically aligned.

2. Device according to claim 1, characterised in that, in addition, it comprises a diaphragm and/or a second optical lens between the source of light (1) and the chamber (2).

3. Device according to one of the claims 1 and 2, characterised in that the source of light (1) is situated inside the chamber (2).

4. Device according to one of the claims 1 to 3, characterised in that the source of light (1), the porous support (4), the photo-electric receiver (5) and the first optical lens (7) are not geometrically aligned and in that the device comprises a reflexion element in the path of the light bundle, originating from the source (1), before or after its screening by the material (6).

5. Device according to one of the claims 1 to 4, characterised in that the photo-electric receiver (5) is a photomultiplier tube.

6. Device according to one of the claims 1 to 4, characterised in that the photo-electric receiver (5) is a bar of microphotodiodes.

7. Device according to one of the claims 1 to 6, characterised in that the photo-electric receiver (5) is mounted so as to be transversely movable with respect to the light bundle originating from the source of light (1).

8. Device according to one of the claims 1 to 7, characterised in that the porous support (4) is a bed of coke powder, contained in a boat.

9. Device according to one of the claims 1 to 7, characterised in that the porous support (4) is a sintered glass pellet.

10. Application of the device according to one of the claims 1 to 9 to the determination of the wetting power of pitches, on the basis of the tracing of the contour of these pitches in the course of deformation.

## Ansprüche

1. Einrichtung zur Messung der Verformung eines Materials (6) unter Wärmeeinwirkung, dadurch gekennzeichnet, daß sie
— eine Lichtquelle (1),
— eine zumindest teilweise transparente Einfassung (2), die mit einer zeitabhängig programmierbaren Heizeinrichtung (3) und einem zur Aufnahme des Materials (6) bestimmten porösen Träger (4) ausgestattet ist,
— einen photoelektrischen Empfänger (5) und
— eine erste optische Linse (7), die die Bildung des Bildes des Materials (6) auf dem photoelektrischen Empfänger (5) erlaubt, enthält, wobei die Lichtquelle (1), der poröse Träger (4), der photoelektrische Empfänger (5) und die erste optische Linse (7) optisch ausgerichtet sind.

2. Einrichtung nach Anspruch 1, dadurch gekennzeichnet, daß sie weiters zwischen der Lichtquelle (1) und der Einfassung (2) eine Blende und/oder eine zweite optische Linse enthält.

3. Einrichtung nach Anspruch 1 oder 2, dadurch gekennzeichnet, daß die Lichtquelle (1) im Inneren der Einfassung (2) angeordnet ist.

4. Einrichtung nach einem der Ansprüche 1 bis 3, dadurch gekennzeichnet, daß die Lichtquelle (1), der poröse Träger (4), der photoelektrische Empfänger (5) und die erste optische Linse (7) nicht geometrisch ausgerichtet sind, und daß die Einrichtung ein Reflektororgan im Weg des von der Quelle (1) kommenden Lichtbündels vor oder nach dessen Verdunkelung durch das Material (6) enthält.

5. Einrichtung nach einem der Ansprüche 1 bis 4, dadurch gekennzeichnet, daß der photoelektrische Empfänger (5) eine Photovervielfacherröhre ist.

6. Einrichtung nach einem der Ansprüche 1 bis 4, dadurch gekennzeichnet, daß der photoelektrische Empfänger (5) eine Mikrophotodiodenreihe ist.

7. Einrichtung nach einem der Ansprüche 1 bis 6, dadurch gekennzeichnet, daß der photoelektrische Empfänger (5) in bezug auf das von der Lichtquelle (1) kommende Lichtbündel querbeweglich montiert ist.

8. Einrichtung nach einem der Ansprüche 1 bis 7, dadurch gekennzeichnet, daß der poröse Träger (4) ein in einem Schiffchen enthaltenes Kokspulverbett ist.

9. Einrichtung nach einem der Ansprüche 1 bis 7, dadurch gekennzeichnet, daß der poröse Träger (4) eine Scheibe aus Sinterglas ist.

10. Anwendung der Einrichtung nach einem der Ansprüche 1 bis 9 zur Bestimmung des Erweichungsvermögens von Pechen auf Basis der Verfolgung der Kontur dieser Peche während der Verformung.

FIGURE 1.

FIGURE 2.

FIGURE 3.